Europäisches Patentamt

European Patent Office

Office européen des brevets

(11) Numéro de publication: **0 461 975 B1**

# FASCICULE DE BREVET EUROPEEN

(45) Date de publication de fascicule du brevet: **15.02.95**

(51) Int. Cl.6: **C07C 69/54**, C07C 67/08, C07C 67/03

(21) Numéro de dépôt: **91401515.1**

(22) Date de dépôt: **10.06.91**

(54) **La préparation d'esters d'acides carboxyliques insaturés par (trans)estérification en phase liquide en utilisant des hétéropolyacides comme catalyseurs.**

(30) Priorité: **13.06.90 FR 9007368**

(43) Date de publication de la demande:
**18.12.91 Bulletin 91/51**

(45) Mention de la délivrance du brevet:
**15.02.95 Bulletin 95/07**

(84) Etats contractants désignés:
**AT BE CH DE DK ES FR GB GR IT LI LU NL SE**

(56) Documents cités:
**GB-A- 458 236**
**US-A- 3 442 934**
**US-A- 3 442 935**

(73) Titulaire: **ELF ATOCHEM S.A.**
**4 & 8, Cours Michelet**
**La Défense 10**
**F-92800 Puteaux (FR)**

(72) Inventeur: **Paumard, Eric**
**338, rue de l'Abbé Touba**
**F-57450 Cappel (FR)**

## Description

La présente invention porte sur l'utilisation d'hétéropolyacides comme catalyseurs dans la préparation en phase liquide d'esters d'acides carboxyliques insaturés, notamment l'acide acrylique et l'acide méthacrylique, par estérification directe de ces acides par des alcools, ou encore par transestérification, ainsi que sur le procédé de préparation correspondant.

Dans les réactions du type indiqué ci-dessus, qui sont avantageusement conduites dans des conditions douces de température (par exemple, à moins de 100°C) ou de pression (pression atmosphérique ou pression réduite) - ce qui permet d'éviter une polymérisation de l'ester au cours de sa préparation -, on utilise généralement un catalyseur acide de type Bronsted ou Lewis, comme l'acide sulfurique ou un titanate d'alkyle. Toutefois, un tel acide pose des problèmes de pollution et de corrosion, et sa désactivation est rapide.

Par ailleurs, dans la demande de brevet japonais n° 175 573/1980, on décrit un procédé de préparation d'un (méth)acrylate, qui comprend la réaction de l'acide (méth)acrylique avec un alcool inférieur, à une température élevée, en phase gazeuse, en présence d'oxygène moléculaire et d'un catalyseur ayant une structure d'hétéropolyacide, contenant du phosphore et du molybdène.

En outre, les hétéropolyacides sont connus pour être utilisés comme catalyseurs dans la réaction d'oléfines sur les acides, par exemple de l'éthylène sur l'acide acrylique, comme cela est décrit dans le brevet français n° 2 404 621, la réaction étant effectuée à température et pression élevées.

La Société Déposante a maintenant constaté que l'utilisation d'hétéropolyacides dans la préparation en phase liquide d'esters d'acides carboxyliques insaturés par estérification ou transestérification permet d'inhiber la formation de sous-produits, conduisant aux esters visés avec des taux de conversion élevés et des sélectivités élevées, en supprimant tous les inconvénients de l'acide sulfurique. C'est ainsi notamment que les temps de réaction sont plus courts, et que les problèmes de pollution et de corrosion sont supprimés. De plus, les hétéropolyacides constituent des catalyseurs en milieu homogène qui sont recyclables dans des procédés en discontinu.

La présente invention a donc d'abord pour objet l'utilisation d'au moins un hétéropolyacide de formule générale :

$$H_n A_a D_c O_y . x H_2 O$$

dans laquelle :
- A représente le phosphore, le bore, le silicium, le germanium, l'étain, l'arsenic, l'antimoine, le cuivre, le nickel, le cobalt, le fer, le cérium, le thorium, le chrome ou un mélange d'au moins deux de ces éléments ;
- D représente le molybdène, le tungstène, le vanadium ou une combinaison d'au moins deux de ces éléments ;
- a est un nombre de 0,1 à 10 ;
- c est un nombre de 6 à 18 ;
- n est le nombre d'hydrogènes acides dans l'hétéropolyacide et est un nombre supérieur à 1 ;
- y est le nombre d'oxygènes dans l'hétéropolyacide et est un nombre de l'ordre de 10 à 70 ; et
- x est le nombre de moles d'eau de cristallisation et est un nombre de 0 à environ 40,

comme catalyseur dans la préparation d'esters d'acides carboxyliques insaturés par estérification directe desdits acides par des alcools ou par transestérification, ces réactions étant conduites en phase liquide.

La présente invention a également pour objet un procédé de préparation d'un ester d'acide carboxylique insaturé par une réaction en phase liquide d'estérification directe dudit acide par un alcool ou par une réaction de transestérification, en présence d'un catalyseur acide, caractérisé par le fait qu'on utilise, comme catalyseur, au moins un hétéropolyacide de la formule générale indiquée ci-dessus.

Ces hétéropolyacides sont connus et ils peuvent être préparés par des techniques connues. Des hétéropolyacides particulièrement intéressants sont l'acide phosphomolybdique ($H_3 PMo_{12} O_{40}$), l'acide phosphotungstique ($H_3 PW_{12} O_{40}$) et l'acide silicotungstique ($H_4 SiW_{12} O_{40}$).

Les catalyseurs utilisés dans la réaction sont utilisés d'une façon appropriée comme composants à l'état dissous d'un mélange liquide. On obtient des résultats intéressants, suivant la présente invention, avec le mélange liquide homogène à cause de l'efficacité de contact sensiblement supérieure.

La concentration du catalyseur selon l'invention dans le mélange de réaction en phase liquide peut varier largement. Pour de tels catalyseurs homogènes, il est plus approprié d'établir la concentration en catalyseur en fonction de la concentration du réactif minoritaire en mole par mole d'acide ou en mole par mole d'alcool. On a ainsi constaté qu'il est préférable d'utiliser des concentrations molaires d'environ $1.10^{-3}$

à 5.10$^{-2}$.

Les conditions opératoires pour les réactions d'estérification et de transestérification en phase liquide peuvent varier largement ; cependant, dans la pratique normale, la température peut se situer entre 20° et 200°C, et la réaction est normalement réalisée à la pression atmosphérique ou sous une pression inférieure à la pression atmosphérique. Se révèlent particulièrement intéressantes dans le cadre du procédé de la présente invention les réactions réalisées à des températures de 60° à 130°C, en utilisant une pression inférieure à la pression atmosphérique, par exemple, inférieure à 6 x 10$^4$ Pa. La durée de la réaction peut varier également dans de larges limites ; elle peut être courte, par exemple, de quelques minutes, ou être de plusieurs heures suivant l'état du catalyseur utilisé, la température de réaction et la pression. Le rapport molaire de l'alcool à l'acide ou à l'ester peut varier pour autant que le produit prédominant soit l'ester visé. D'une façon appropriée, les rapports molaires sont d'environ 0,1 à environ 3 moles de l'alcool par mole de l'acide ou de l'ester de départ.

L'alcool utilisé pour la réaction d'estérification ou de transestérification sera, par exemple, un monoalcool saturé, primaire, secondaire ou tertiaire, ayant de 1 à 22 atomes de carbone, comme le propanol, le méthanol, l'éthanol, le n-butanol, l'isopropanol, le sec-butanol, le tertiobutanol, le 2-éthylhexanol ; un éther-alcool de formule $C_nH_{2n+1}$-O-$C_nH_{2n+1}$-OH dans laquelle n est un nombre entier allant de 1 à 5 comme le méthoxyéthanol, l'éthoxyéthanol, le méthoxyméthanol et l'éthoxyéthanol ; un polyol comme l'éthylèneglycol, le propylèneglycol, le 1,3-butanediol, le 1,4-butanediol, le 1,6-hexanediol, le néopentylglycol, le 1,4-cyclohexanediol, le 1,4-cyclohexanediméthanol, le 2,2,4-triméthyl-1,3-pentanediol, le 2-éthyl-2-méthyl-1,3-propanediol, le 2,2-diéthyl-1,3-propanediol, le diéthylèneglycol, le dipropylèneglycol, le triéthylèneglycol, le tripropylèneglycol, le tétraéthylèneglycol, le tétrapropylèneglycol, le triméthyloléthane, le triméthylolpropane, le glycérol, le pentaérythritol, et les polyols mono- ou polyéthoxylés et mono- ou polypropoxylés.

L'utilisation d'inhibiteurs de polymérisation est généralement souhaitée pour éviter que les esters et/ou acides insaturés présents ne polymérisent. On utilise à cet effet, de préférence, des phénols comme l'hydroquinone, l'éther méthylique de l'hydroquinone, le di-tertiobutylcatéchol ou le p-anilinophénol, des amines aromatiques en présence d'oxygène, la N,N-diéthylhydroxylamine, le nitrobenzène, la phénothiazine, le phosphite de di-(2-éthyl-hexyl)-octylphényle, le bleu de méthylène et leurs mélanges en toutes proportions. Les inhibiteurs de polymérisation sont généralement utilisés à raison de 0,05-1% en poids par rapport au poids total des réactifs engagés dans la réaction.

Par ailleurs, on peut effectuer la réaction selon l'invention en présence d'un solvant inerte capable de jouer la fonction d'agent azéotropique de l'eau formée dans la réaction. L'hétéroazéotrope formé entre l'eau et ledit solvant décante. La phase aqueuse est recueillie et le solvant présent dans la phase organique est renvoyé à la réaction. Le solvant peut être choisi notamment parmi :
- les alcools saturés avant de 1 à 4 atomes de carbone,
- les hydrocarbures aromatiques légers tels que le benzène,
- les cétones aliphatiques telles que la méthylisobutylcétone,
- les alcanes linéaires et cycliques ayant 6 ou 7 atomes de carbone, comme l'hexane, le cyclohexane, l'heptane et le méthylcyclohexane.

Le solvant est généralement utilisé à raison de jusqu'à 50% environ en poids dans le milieu réactionnel (réactifs + solvant).

Les réactions selon l'invention sont réalisées avantageusement dans un réacteur convenant pour une réaction en phase liquide. C'est ainsi que l'on peut utiliser un récipient agité ou non et chauffé contenant les réactifs avec le catalyseur dissous dans ceux-ci. Dans une opération discontinue, les produits sont séparés par distillation fractionnée avec des colonnes de distillation. L'anion de l'hétéropolyacide ayant tendance à se décomposer au contact d'ions de métaux lourds tels que le fer et le nickel, il est donc souhaitable que le matériau constituant le réacteur ne libère pas de tels ions de métaux. On pourra utiliser par exemple un réacteur en acier émaillé.

En outre, conformément à l'invention, à la fin de la réaction d'estérification ou de transestérification, on peut soumettre le produit brut réactionnel à une distillation, récupérer le résidu contenant le catalyseur et, le cas échéant, le ou les inhibiteurs de polymérisation, que l'on réutilise dans une nouvelle opération d'estérification ou de transestérification, un tel recyclage du catalyseur pouvant avoir lieu plusieurs fois.

Les exemples suivants illustrent la présente invention, sans toutefois en limiter la portée. D'après les résultats de l'analyse du produit de réaction de chaque exemple, on détermine la composition du produit, puis les performances du catalyseur, par les trois critères de conversion, sélectivité et rendement, définis de la façon suivante :
(a) dans le cas d'une estérification directe

% conversion (C) = (moles d'acide ayant réagi/moles d'acide chargées) x 100

EP 0 461 975 B1

% sélectivité (S) = (moles d'ester obtenues/moles d'acide ayant réagi) x 100
% rendement (R) = (moles d'ester obtenues/moles d'acide chargées) x 100

(b) dans le cas d'une transestérification

% conversion (C) = (moles d'alcool ayant réagi/moles d'alcool chargées) x 100
% sélectivité (S) = (moles d'ester obtenues/moles d'alcool ayant réagi) x 100
% rendement (R) = (moles d'ester obtenues/moles d'alcool chargées) x 100

EXEMPLE 1 : Transestérification entre l'acrylate d'éthyle et le butanol

Dans un réacteur autoclave de verre de 1 litre, équipé d'un agitateur mécanique et surmonté d'une colonne à reflux, d'un condenseur et d'un collecteur, on place 550 g (5,5 moles) d'acrylate d'éthyle, 220 g (3 moles) de butanol, et 21 g de catalyseur $H_3PW_{12}O_{40}$ (7,25 x $10^{-3}$ mole), soit 2,4 x $10^{-3}$ mole par mole de butanol. On introduit 1,5 g d'hydroquinone (0,2% en poids par rapport à la charge des réactifs). Tout en agitant, on abaisse la pression à 6 x $10^4$ Pa et on porte la temperature à 95°C au moyen d'une enveloppe chauffante. On conduit alors la réaction de l'alcool sur l'ester léger ; au cours de la réaction, on élimine l'éthanol libéré sous la forme d'un azéotrope avec l'acrylate d'éthyle. Après 5 heures de réaction, on refroidit le mélange à température ambiante, et on analyse le produit par chromatographie en phase gazeuse. Les résultats sont présentés au Tableau 1.

EXEMPLE 2 : Transestérification entre le méthacrylate de méthyle et le butanol

Dans le même appareil que celui utilisé à l'Exemple 1, on fait réagir 550 g (5,5 moles) de méthacrylate de méthyle avec le butanol, dans les mêmes conditions que dans cet exemple, à ceci près que l'on conduit la réaction pendant 7 heures.
Les résultats sont indiqués au Tableau 1.

TABLEAU 1

| Exemple | C | S | R |
|---------|-----|-----|------|
| 1 | 98 | 73 | 71,5 |
| 2 | 82 | 95 | 78 |

EXEMPLE 3 : Estérification directe de l'acide acrylique par le butanol

Dans le même appareil que celui utilisé à l'Exemple 1 , on place 216 g (3 moles) d'acide acrylique, 290 g (3,9 moles de butanol) et 21 g de catalyseur $H_3PW_{12}O_{40}$ (7,2 x $10^{-3}$ mole), soit 2,4 x $10^{-3}$ mole par mole d'acide acrylique. On introduit 0,1% en poids d'hydroquinone et 0,1% en poids de phénothiazine par rapport à la charge des réactifs. On procède comme à l'Exemple 1, mais en utilisant une température de réaction de 90°C et une pression de 1,5 x $10^4$ Pa. La réaction est conduite pendant 3 heures.
Les résultats sont indiqués au Tableau 2.

EXEMPLES 4 et 5

Après distillation des réactifs n'ayant pas réagi du produit brut de l'Exemple 3 et de l'acrylate de butyle, on place une nouvelle charge identique d'alcool et d'acide sur le résidu de distillation contenant le catalyseur et les inhibiteurs de polymérisation, et on conduit la réaction dans les mêmes conditions (Exemple 4).
On recommence une nouvelle fois cette même opération (Exemple 5) à partir du résidu de distillation de l'Exemple 4.
Les résultats sont indiqués au Tableau 2.

4

EXEMPLES 6 et 7

On reproduit les Exemples respectivement 3 et 4 dans les mêmes conditions, excepté que le catalyseur est remplacé par $H_3PMo_{12}O_{40}$ et que l'on conduit la réaction pendant 2,5 heures à l'Exemple 6, et pendant 2 heures à l'Exemple 7. Les résultats sont également indiqués au Tableau 2.

TABLEAU 2

| Exemple | Nature du catalyseur | C | R | S |
|---|---|---|---|---|
| 3 | $H_3PW_{12}O_{40}$ | 99 | 92,4 | 93,3 |
| 4 | Recyclage du résidu de l'Exemple 3 | 98 | 85,6 | 87,3 |
| 5 | Recyclage du résidu de l'Exemple 4 | 97 | 80,5 | 83 |
| 6 | $H_3PMo_{12}O_{40}$ | 99 | 90 | 91 |
| 7 | Recyclage du résidu de l'Exemple 6 | 97 | 84,4 | 87 |

EXEMPLE 8 à 11 : Estérification directe de l'acide acrylique par le butanol avec différents catalyseurs

On effectue la réaction dans un réacteur de 1 litre, non agité mécaniquement, pourvu d'une colonne à reflux, d'un condenseur et d'un collecteur, de sorte qu'au fur et mesure que la réaction progresse, le produit le plus volatil, à savoir l'eau de réaction, est séparé par distillation au fur et à mesure qu'il se forme.

Le protocole expérimental est le même qu'à l'Exemple 3, avec une charge de 3 moles d'acide acrylique et de 3,9 moles de butanol, à laquelle on ajoute $2,5 \times 10^{-3}$ mole de catalyseur par mole d'acide, et, comme inhibiteurs de polymérisation, 0,1% en poids d'hydroquinone et 0,1% en poids de phénothiazine par rapport à la charge des réactifs, le temps de réaction figurant dans le Tableau 3.

TABLEAU 3

| Exemple | Nature du catalyseur | Temps de réaction (mn) | R | C | S |
|---|---|---|---|---|---|
| 8 | $H_3PW_{12}O_{40}$ | 255 | 93,6 | 98 | 95,6 |
| 9 | $H_3PMo_{12}O_{40}$ | 315 | 89,7 | 92,8 | 96,7 |
| 10 | $H_4SiW_{12}O_{40}$ | 200 | 93,3 | 96,3 | 97 |
| 11 (comparatif) | $H_2SO_4$ | 670 | 86,9 | 98,1 | 89 |

Les résultats montrent une nettement plus grande réactivité des catalyseurs de l'invention par comparaison avec l'acide sulfurique.

EXEMPLES 12 à 21 : Estérification directe d'acide (méth) acrylique par différents alcools avec $H_4SiW_{12}O_{40}$ comme catalyseur

On procède comme pour les Exemples 8 à 11, avec une charge de 3 moles d'acide sauf pour les Exemples 15 et 16 (2 moles), un rapport molaire alcool/acide tel qu'indiqué au Tableau 4, et la présence éventuelle d'un solvant azéotropique, à raison de 30% en poids de la charge, pour entraîner l'eau. Le catalyseur est utilisé à raison de $2,5 \times 10^{-3}$ mole par mole d'acide. Les températures, pressions et temps de réaction et les résultats sont présentés au Tableau 4 (pour les Exemples 17, 18 et 21, la sélectivité indiquée est celle correspondant à la somme des mono-, di- et triacrylates formés).

TABLEAU 4

| Exemple | Alcool | Acide | Temps de réaction (mn) | Rapport molaire alcool/acide | solvant azéotropique | T °C | P (10⁴Pa) | R | C | S |
|---|---|---|---|---|---|---|---|---|---|---|
| 12 | butanol | AMA | 570 | 1,3 | butanol | 80 | 1,3 | 94,4 | 96,2 | 98,8 |
| 13 | éthyl-2hexanol | AA | 350 | 1,1 | MIBC | 90 | 1 | 93,5 | 96 | 97,3 |
| 14 | " | AMA | 320 | 1,1 | MIBC | 95 | 1 | 84 | 86,8 | 97 |
| 15 | alcool laurylique | AA | 240 | 1,1 | heptane | 81 | 2,5 | 90,5 | 98,4 | 92 |
| 16 | " | AMA | 330 | 1,1 | " | 95 | 2,5 | 91,5 | 99,3 | 92,1 |
| 17 | éthylène glycol | AA | 350 | 0,47 | " | 85 | 3 | 50 | 87,4 | 57(a) |
| 18 | " | AMA | 450 | 0,47 | " | 80 | 2,5 | 20 | 59 | 34(b) |
| 19 | méthoxyéthanol | AA | 270 | 1,1 | benzène | 80 | 4,2 | 89 | 92 | 97 |
| 20 | " | AMA | 420 | 1,1 | " | 83 | 4,2 | 83,7 | 78,1 | 99 |
| 21 | tripropylène glycol | AA | 1000 | 0,46 | heptane | 80 | 4,7 | 25 | 55 | 46(c) |

AMA : acide méthacrylique
AA : acide acrylique
MIBC : méthylisobutylcétone

(a) dont 39% de diacrylate et 18% de monoacrylate
(b) dont 26% de diacrylate et 8% de monoacrylate
(c) dont 25,5% de diacrylate du tripropylène glycol, 10,5% de monoacrylate du tripropylèneglycol et 10% de diacrylate du dipropylèneglycol

## Revendications

1. Procédé de préparation d'un ester d'acide carboxylique à insaturation $\alpha,\beta$ éthylénique par une reaction en phase liquide d'estérification directe dudit acide par un alcool ou par une réaction de transestérifica-

6

tion, en présence d'un catalyseur acide, caractérisé par le fait qu'on utilise, comme catalyseur, au moins un hétéropolyacide de formule générale :

$$H_nA_aD_cO_y.xH_2O$$

dans laquelle :
- A représente le phosphore, le bore, le silicium, le germanium, l'étain, l'arsenic, l'antimoine, le cuivre, le nickel, le cobalt, le fer, le cérium, le thorium, le chrome ou un mélange d'au moins deux de ces éléments ;
- D représente le molybdène, le tungstène, le vanadium ou une combinaison d'au moins deux de ces éléments ;
- a est un nombre de 0,1 à 10 ;
- c est un nombre de 6 à 18 ;
- n est le nombre d'hydrogènes acides dans l'hétéropolyacide et est un nombre supérieur à 1 ;
- y est le nombre d'oxygènes dans l'hétéropolyacide et est un nombre de 10 à 70 ; et
- x est le nombre de moles d'eau de cristallisation et est un nombre de 0 à 40.

2. Procédé selon la revendication 1, caractérisé par le fait qu'on utilise, comme hétéropolyacide, au moins l'un parmi l'acide phosphomolybdique, l'acide phosphotungstique ou l'acide silicotungstique.

3. Procédé selon l'une des revendications 1 et 2, caractérisé par le fait qu'on utilise le catalyseur à raison de $1 \times 10^{-3}$ à $5 \times 10^{-2}$ mole par mole du réactif minoritaire.

4. Procédé selon l'une des revendications 1 à 3, caractérisé par le fait qu'on conduit la réaction à une température comprise entre 20°C et 200°C, à la pression atmosphérique ou sous une pression inférieure à la pression atmosphérique.

5. Procédé selon l'une des revendications 1 à 4, caractérisé par le fait qu'on utilise un rapport molaire de 0,1:1 à 3:1 de l'alcool à l'acide ou à l'ester de départ.

6. Procédé selon l'une des revendications 1 à 5, caractérisé par le fait qu'on utilise, comme acide de départ dans l'estérification directe, l'acide acrylique ou l'acide méthacrylique, et, comme alcool, un monoalcool saturé ayant de 1 jusqu'à 22 atomes de carbone, un éther-alcool de formule $C_nH_{2n+1}OC_nH_{2n+1}OH$ dans laquelle n est un nombre entier allant de 1 à 5, un polyol, un polyol mono- ou polyéthoxylé ou un polyol mono- ou polypropoxylé.

7. Procédé selon l'une des revendications 1 à 6, caractérisé par le fait qu'on conduit la réaction en présence d'au moins un inhibiteur de polymérisation à raison de 0,05-1% en poids par rapport au poids total des réactifs.

8. Procédé selon l'une des revendications 1 à 7, caractérisé par le fait qu'on conduit la réaction en présence d'un solvant inerte.

9. Procédé selon l'une des revendications 1 à 8, caractérisé par le fait qu'à la fin de la réaction, on soumet le produit brut réactionnel à une distillation, on récupère un résidu contenant le catalyseur et, le cas échéant, le ou les inhibiteurs de polymérisation, que l'on réutilise dans une nouvelle opération d'estérification ou de transestérification, un tel recyclage du catalyseur pouvant avoir lieu plusieurs fois.

10. Procédé selon la revendication 8, caractérisé par le fait que le solvant inerte est choisi parmi :
- les alcools saturés ayant de 1 à 4 atomes de carbone,
- les hydrocarbures aromatiques légers,
- les cétones aliphatiques, et
- les alcanes linéaires et cycliques ayant 6 ou 7 atomes de carbone.

11. Procédé selon l'une des revendications 8 et 10, caractérisé par le fait que le solvant est utilisé à raison de jusqu'à 50% en poids dans le milieu réactionnel.

**Claims**

1.  Process for the preparation of an $\alpha,\beta$-ethylenically unsaturated carboxylic acid ester by a direct liquid-phase esterification reaction of said acid with an alcohol or by a transesterification reaction in the presence of an acid catalyst, characterized in that the catalyst used is at least one heteropolyacid of the general formula:

    $$H_nA_aD_cO_y \bullet xH_2O$$

    in which:
    -   A represents phosphorus, boron, silicon, germanium, tin, arsenic, antimony, copper, nickel, cobalt, iron, cerium, thorium, chromium or a mixture of at least two of these elements;
    -   D represents molybdenum, tungsten, vanadium or a combination of at least two of these elements;
    -   a is a number from 0.1 to 10;
    -   c is a number from 6 to 18;
    -   n is the number of acidic hydrogens in the heteropolyacid and is a number above 1;
    -   y is the number of oxygens in the heteropolyacid and is a number from 10 to 70; and
    -   x is the number of moles of water of crystallization and is a number from 0 to 40.

2.  Process according to Claim 1, characterized in that the heteropolyacid used is at least one selected from phosphomolybdic acid, phosphotungstic acid or silicotungstic acid.

3.  Process according to one of Claims 1 and 2, characterized in that the catalyst is used in an amount of $1 \times 10^{-3}$ to $5 \times 10^{-2}$ mol per mole of minor reactant.

4.  Process according to one of Claims 1 to 3, characterized in that the reaction is carried out at a temperature between 20°C and 200°C, at atmospheric pressure or under a pressure below atmospheric pressure.

5.  Process according to one of Claims 1 to 4, characterized in that a molar ratio of alcohol to starting acid or ester of 0.1:1 to 3:1 is used.

6.  Process according to one of Claims 1 to 5, characterized in that the starting acid used in the direct esterification is acrylic acid or methacrylic acid and the alcohol used is a saturated monoalcohol having 1 to 22 carbon atoms, an ether alcohol of the formula $C_nH_{2n+1}OC_nH_{2n+1}OH$ in which n is an integer ranging from 1 to 5, a polyol, a mono- or polyethoxylated polyol or a mono- or polypropoxylated polyol.

7.  Process according to one of Claims 1 to 6, characterized in that the reaction is carried out in the presence of at least one polymerization inhibitor in an amount of 0.05-1% by weight, relative to the total weight of the reactants.

8.  Process according to one of Claims 1 to 7, characterized in that the reaction is carried out in the presence of an inert solvent.

9.  Process according to one of Claims 1 to 8, characterized in that at the end of the reaction the crude reaction product is subjected to distillation, and the residue containing the catalyst and, if present, the polymerization inhibitor(s), which is(are) reused in a new esterification or transesterification operation, are recovered, it being possible for such a recycling of the catalyst to take place several times.

10. Process according to Claim 8, characterized in that the inert solvent is chosen from:
    -   saturated alcohols having 1 to 4 carbon atoms,
    -   light aromatic hydrocarbons,
    -   aliphatic ketones, and
    -   linear and cyclic alkanes having 6 or 7 carbon atoms.

11. Process according to one of Claims 8 and 10, characterized in that the solvent is used in an amount of up to 50% by weight in the reaction medium.

8

**Patentansprüche**

1. Verfahren zur Herstellung von $\alpha,\beta$-ethylenisch ungesättigten Carbonsäureestern durch direkte Veresterung der Säure mit einem Alkohol in flüssiger Phase oder durch Umesterung in Gegenwart eines sauren
Katalysators,
gekennzeichnet durch
die Verwendung von mindestens einer Heteropolysäure der allgemeinen Formel

   $H_nA_aD_cO_y \cdot x\ H_2O$

   als Katalysator, worin bedeuten:
   - A Phosphor, Bor, Silicium, Germanium, Zinn, Arsen, Antimon, Kupfer, Nickel, Cobalt, Eisen, Cer, Thorium, Chrom oder ein Gemisch von mindestens zwei dieser Elemente,
   - D Molybdän, Wolfram, Vanadium oder eine Kombination von mindestens zwei dieser Elemente,
   - a eine Zahl von 0,1 bis 10,
   - c eine Zahl von 6 bis 18,
   - n die Anzahl an sauren Wasserstoffatomen in der Heteropolysäure, die größer als 1 ist,
   - y die Anzahl an Sauerstoffatomen in der Heteropolysäure, die 10 bis 70 beträgt, und
   - x die Anzahl an das Kristallwasser bildenden Wassermolekülen, die 0 bis 40 beträgt.

2. Verfahren nach Anspruch 1,
   dadurch gekennzeichnet, daß
   als Heteropolysäure Molybdatophosphorsäure und/oder Wolframatophosphorsäure und/oder Wolframatokieselsäure verwendet wird.

3. Verfahren nach einem der Ansprüche 1 und 2,
   dadurch gekennzeichnet, daß
   der Katalysator in einer Menge von $1 \cdot 10^{-3}$ bis $5 \cdot 10^{-2}$ mol pro Mol des im kleineren Anteil verwendeten Reagens verwendet wird.

4. Verfahren nach einem der Ansprüche 1 bis 3,
   dadurch gekennzeichnet, daß
   die Umsetzung bei einer Temperatur von 20 bis 200 °C, bei Atmosphärendruck oder unter vermindertem Druck durchgeführt wird.

5. Verfahren nach einem der Ansprüche 1 bis 4,
   dadurch gekennzeichnet, daß
   der Alkohol und die Ausgangssäure bzw. der Ausgangsester in einem Molverhältnis von 0,1:1 bis 3:1 verwendet werden.

6. Verfahren nach einem der Ansprüche 1 bis 5,
   dadurch gekennzeichnet, daß
   bei der direkten Veresterung als Ausgangssäure Acrylsäure oder Methacrylsäure und als Alkohol ein gesättigter Monoalkohol mit 1 bis 22 Kohlenstoffatomen, ein Etheralkohol der Formel $C_nH_{2n+1}OC_nH_{2n+1}OH$, in der n eine ganze Zahl von 1 bis 5 darstellt, ein Polyol, ein mono- oder polyethoxyliertes Polyol oder ein mono- oder polypropoxyliertes Polyol verwendet werden.

7. Verfahren nach einem der Ansprüche 1 bis 6,
   dadurch gekennzeichnet, daß
   die Umsetzung in Gegenwart von mindestens einem Polymerisationsinhibitor in einer Menge von 0,05 bis 1 Gew.-%, bezogen auf das Gesamtgewicht der Reagentien, durchgeführt wird.

8. Verfahren nach einem der Ansprüche 1 bis 7,
   dadurch gekennzeichnet, daß
   die Umsetzung in Gegenwart eines inerten Lösungsmittels durchgeführt wird.

9. Verfahren nach einem der Ansprüche 1 bis 8,
   dadurch gekennzeichnet, daß
   am Ende der Umsetzung das Rohprodukt dieser Umsetzung einer Destillation unterzogen und ein Rückstand gewonnen wird, der den Katalysator und ggfs. den oder die Polymerisationsinhibitoren enthält, die bei weiteren Arbeitsgängen der Veresterung oder Umesterung wiederverwendet werden, wobei eine solche Wiederverwertung des Katalysators mehrfach stattfinden kann.

10. Verfahren nach Anspruch 8,
    dadurch gekennzeichnet, daß
    das inerte Lösungsmittel unter
    - gesättigten Alkoholen mit 1 bis 4 Kohlenstoffatomen,
    - leichten aromatischen Kohlenwasserstoffen,
    - aliphatischen Ketonen und
    - geradkettigen und cyclischen Alkanen mit 6 oder 7 Kohlenstoffatomen
    ausgewählt ist.

11. Verfahren nach einem der Ansprüche 8 bis 10,
    dadurch gekennzeichnet, daß
    das Lösungsmittel in einer Menge von bis zu 50 Gew.-% im Reaktionsmedium verwendet wird.